# FASCICULE DE BREVET EUROPEEN

(11) **EP 2 584 355 B1**
(45) Date de publication et mention de la délivrance du brevet: **16.09.2015**
(21) Numéro de dépôt: 12189235.0
(22) Date de dépôt: 19.10.2012
(51) Int. Cl.: G01N 33/18, G01N 1/16

(54) **Dispositif d'acquisition pour la réalisation de mesures et/ou le prélèvement d'échantillons dans un liquide**
Erfassungsvorrichtung für die Durchführung von Messungen und/oder Probenentnahmen in einer Flüssigkeit
Acquisition device for taking measurements and/or collecting samples from a liquid

(30) Priorité: 20.10.2011 FR 1159526
(43) Date de publication de la demande: 24.04.2013
(73) Titulaire: Institut Français des Sciences et Technologies des Transports, de l'Aménagement et des Réseaux, 77420 Champs sur Marne (FR); Ecole Nationale des Ponts et Chaussées, 77420 Champs-Sur-Marne (FR); Institut De Physique Du Globe De Paris (Établissement Public À Caractère Scientifique Et Technologique), 75005 Paris (FR)
(72) Inventeur: Derkx, François, 92120 Montrouge (FR); Merliot, Erick, 78650 Beynes (FR); Sorin, Jean-Luc, 44700 Orvault (FR); Tassin, Bruno, 94300 Vincennes (FR); Prevot, François, 94420 Le Plessis Trevise (FR)
(74) Mandataire: Intès, Didier Gérard André

(56) Documents cités:
- DE-A1- 10 015 587
- US-B1- 7 559 236
- US-B1- 7 690 247
- Tufts University: "Automated buoy", Tufts University Center for Engineering Educational Oureach, 1 octobre 2005 (2005-10-01), XP055044558, Extrait de l'Internet: URL:http://www.ceeo.tufts.edu/underwater/A utomated Buoy.pdf [extrait le 2012-11-16]
- KEN LI ET AL: "Remote controlled helicopters: a tool for air sampling in difficult situations", JOURNAL OF HAZARDOUS MATERIALS, vol. 43, 1 janvier 1995 (1995-01-01), pages 117-127, XP055027719,
- Volker Bertram: "Unmanned Surface Vehicles - A Survey", , 1 octobre 2008 (2008-10-01), pages 1-14, XP055027691, Extrait de l'Internet: URL:http://www.skibstekniskselskab.dk/publ ic/dokumenter/Skibsteknisk/Download materiale/2008/10 marts 08/USVsurvey_DTU.pdf [extrait le 2012-05-21]

## Description

L'invention concerne un dispositif d'acquisition comprenant une centrale d'acquisition incluant un ou plusieurs capteur(s) ou système(s) de prélèvement d'échantillon. Ce dispositif permet au cours de phases dites phases d'acquisition, pendant lesquelles la centrale est immergée au moins en partie dans un liquide, d'effectuer la mesure de propriété(s) du liquide à l'aide du ou des capteurs, et/ou de prélever des échantillons du liquide à l'aide du ou des système(s) de prélèvement d'échantillon, et cela à différentes profondeurs sous la surface du liquide.

Le liquide dont les propriétés sont mesurées ou qui est échantillonné est généralement l'eau d'un plan d'eau ou de la mer ; il peut s'agir du liquide constituant une nappe de polluant, par exemple par des hydrocarbures, ou encore tout autre liquide.

La fonction d'un tel dispositif est de collecter des informations ou des échantillons afin de permettre de suivre la qualité du liquide étudié, par exemple la qualité de l'eau du plan d'eau étudié.

En effet, en raison des pollutions et des multiples atteintes aux milieux naturels aquatiques, il est de plus en plus souvent nécessaire de mettre en place des actions de suivi de la qualité des plans d'eau. Le suivi de la qualité d'un plan d'eau consiste habituellement à réaliser périodiquement des mesures et/ou prélèvements d'échantillons afin d'obtenir des informations permettant d'évaluer les changements de la qualité des eaux du ou des plans d'eau. Les mesures effectuées peuvent être de nombreux types. Elles peuvent être des mesures de propriétés relatives au liquide : pH, turbidité, salinité, teneur en tel ou tel composant, température, etc. Elles peuvent être la mesure de paramètres biologiques, chimiques, hydrologiques.

Pour simplifier la description qui suit, le seul liquide mentionné sera l'eau ; cependant ce terme devra être compris comme visant de manière générale tout type de liquide.

Un dispositif permettant le suivi de la qualité des eaux d'un plan d'eau est présenté par le document US2009/0095092. Ce dispositif consiste en un bateau sur lequel est embarquée une centrale d'acquisition permettant le prélèvement d'échantillons.

Cependant, ce bateau est d'un intérêt limité, car il ne permet de réaliser des prélèvements d'échantillon que dans le plan d'eau sur lequel il navigue. Il ne permet donc pas de réaliser des mesures ou des prélèvements d'échantillons répartis sur plusieurs plans d'eau.

Par ailleurs, un autre système de mesure de la qualité de l'eau est présenté par le brevet n°US 7,559,236. Ce brevet présente un dispositif de mesure apte à réaliser des mesures à différentes profondeurs. Dans un mode de réalisation, ce dispositif peut être immergé afin de réaliser des mesures à différentes profondeurs. Pour éviter tout risque d'infiltration d'eau, ce système comporte alors un caisson étanche résistant à la pression, qui accroît nettement le coût du système et rend plus difficile la maintenance de celui-ci.

Le document «Instructor's guide to automated buoy », Tufts University's Center for Engineering Educational Outreach 2005, divulgue une centrale construite en briques Légo à réaliser par les étudiants comme exercice de laboratoire, avec un dispositif de flotteur en couche de polystyrène sur laquelle une structure de surface est simplement posée. Une telle construction n'est pas susceptible d'être employée en mer ou dans un lac.

Aussi, l'objectif de l'invention est de proposer un dispositif d'acquisition avec centrale d'acquisition du type généralement présenté en introduction, permettant non seulement de réaliser des mesures et/ou prélèvements d'échantillons en différents points répartis sur différents plans d'eau, des mesures, et/ou prélèvements d'échantillons, mais de plus de réaliser ces mesures et/ou prélèvements d'échantillons à différentes profondeurs à la surface du liquide ou sous celle-ci.

Cet objectif est atteint grâce à un dispositif d'acquisition comprenant :
- une centrale d'acquisition, comportant :
   - une structure dite structure de surface ;
   - au moins un capteur et/ou au moins un système de prélèvement d'échantillon ; et
   - une liaison mécanique entre ledit au moins un capteur et/ou ledit au moins un système de prélèvement et la structure de surface, agencée de manière à permettre de positionner ledit au moins un capteur et/ou ledit au moins un système de prélèvement à une pluralité de profondeurs dites profondeurs d'acquisition prédéterminées par rapport à une surface d'un liquide ;
- un drone volant, apte à transporter la centrale d'un point à un autre ;
- un dispositif de flotteur apte à maintenir ladite structure de surface en position sensiblement fixe par rapport à la surface du liquide, au moins en partie au-dessus de ladite surface ;
le dispositif étant apte à réaliser une phase d'acquisition pendant laquelle la structure de surface est ainsi maintenue en position sensiblement fixe par rapport à la surface du liquide, et une même mesure d'une propriété relative audit liquide est effectuée par ledit au moins un capteur, et/ou au moins un échantillon du liquide est prélevé par ledit au moins un système de prélèvement d'échantillon, à chacune desdites profondeurs d'acquisition.

Il convient de noter de plus le point suivant. L'indication suivant laquelle ledit au moins un capteur et/ou ledit au moins un système de prélèvement sont positionnés auxdites profondeurs d'acquisition signifie seulement qu'au moins une 'portion de détection' des capteurs - à savoir la partie active des capteurs, grâce à laquelle ceux-ci réalisent leurs mesures -, et/ou les orifices de prélèvement des systèmes de prélèvement d'échantillons - à savoir, les orifices par lesquels les échantillons sont aspirés -, sont positionnés auxdites profondeurs d'acquisition. Cela n'exclut donc pas qu'une portion du ou des capteur(s) et/ou système(s) de prélèvement d'échantillon (i.e., une portion autre que la partie de détection, pour les capteurs, et autre que l'orifice de prélèvement, pour les systèmes de prélèvement d'échantillons) reste à la surface de l'eau, et fasse notamment partie de la structure de surface, en particulier en étant placée à l'intérieur d'une partie aérienne ou émergée de la structure de surface.

Le drone volant est un aéronef sans pilote, télécommandé ou automatique. Il peut être par exemple un avion, un hélicoptère, un ballon dirigeable, etc. Grâce à celui-ci, la centrale peut être déplacée en tout point et notamment d'un plan d'eau à un autre. Le dispositif permet ainsi de réaliser la surveillance d'un ensemble de plans d'eau répartis sur un territoire donné.

D'autre part, le dispositif est agencé de manière à maintenir la centrale d'acquisition sensiblement fixe par rapport au liquide pendant la phase d'acquisition. Grâce à cela, le dispositif permet l'acquisition de mesures et/ou le prélèvement d'échantillons.

Enfin, la liaison mécanique permet de placer le(s) capteur(s) et/ou système(s) de prélèvement d'échantillons aux différentes profondeurs souhaitées. Avantageusement, la liaison mécanique avec la structure de surface permet de positionner de manière simple et précise le(s) capteur(s) et le(s) système de prélèvement d'échantillons aux profondeurs voulues. De plus, une partie des composants de la centrale d'acquisition peut être agencée dans la partie émergée de la structure de surface. Par suite avantageusement, le recours à un caisson étanche peut éventuellement être évité. Par exemple, dans un mode de réalisation la structure de surface peut comporter un boîtier étanche, et le dispositif de flotteur peut être agencé de manière à maintenir ledit boîtier à distance au-dessus de la surface du liquide.

Différents modes de réalisation sont envisageables pour permettre le maintien de la structure de surface en position fixe à la surface du plan d'eau, tout en permettant la réalisation des mesures et/ou prélèvements d'échantillons.

Une double problématique se pose, à savoir la possibilité de déplacements relatifs entre la centrale d'acquisition et le drone, et au sein de la centrale d'acquisition, la possibilité de déplacements relatifs entre la structure de surface et le(s) capteurs et/ou système(s) de prélèvement d'échantillons :

### Déplacements relatifs entre la centrale d'acquisition et le drone

Dans un premier mode de réalisation, la centrale d'acquisition est solidaire du drone et celui-ci est apte à amerrir pour permettre la réalisation de la mesure ou du prélèvement de l'échantillon. L'amerrissage est ici la manoeuvre du drone l'amenant à se poser et s'immobiliser à la surface du liquide, en général donc sur la surface du plan d'eau. Une fois le drone posé sur l'eau, la centrale d'acquisition est (au moins partiellement) immergée, et les mesures et/ou prélèvements d'échantillons peuvent être faits.

Dans ce cas, mais pas nécessairement, le dispositif de flotteur peut être constitué par une partie du drone ; c'est-à-dire qu'il s'agit d'un drone flottant, auquel est fixée la centrale d'acquisition. Le drone lui-même peut alors constituer le dispositif de flotteur au sens de l'invention, assurant le maintien en position à la surface de l'eau (ou au-dessus de la surface) de la structure de surface.

Dans un deuxième mode de réalisation, la centrale d'acquisition est désolidarisée du drone pendant la phase d'acquisition.

Dans ce cas, le dispositif de flotteur est lié à la structure de surface et est mécaniquement indépendant du drone (au moins pendant les phases d'acquisition). Le dispositif de flotteur a alors par lui-même une portance suffisamment grande, par rapport au liquide, pour supporter le poids de l'ensemble de la centrale d'acquisition et rester à la surface de l'eau, et lorsque la centrale d'acquisition est placée dans l'eau ou en surface de l'eau.

Dans une première variante, la centrale reste néanmoins reliée au drone par un câble. Ce câble peut notamment être un câble de treuillage et/ou de transmission d'informations. Dans une seconde variante, la centrale d'acquisition est totalement détachée du drone pendant la phase d'acquisition. L'expression 'totalement détachée' indique ici qu'il n'y a pendant cette phase aucun lien matériel entre la centrale et le drone ; il peut en revanche éventuellement y avoir des communications sans fil. Le dispositif comporte alors des moyens pour permettre la récupération de la centrale à l'issue de la phase d'acquisition, la fixation de celle-ci sur ou au drone pendant le transport vers un nouveau point de mesure ou de prélèvement, et enfin la libération ou le largage de la centrale sur le plan d'eau lorsque le nouveau point auquel des mesures ou prélèvements d'échantillons doivent être faits est atteint.

Le deuxième mode de réalisation précédemment présenté ne nécessite pas l'amerrissage du drone sur le plan d'eau. Cette possibilité est intéressante car elle évite que le drone ne réussisse pas à redécoller du plan d'eau.

La première variante du deuxième mode de réalisation, dans laquelle le drone relié à la centrale par un câble, est particulièrement intéressante car elle résout la difficulté de la récupération de la centrale d'acquisition (problème qui peut survenir dans la deuxième variante de ce mode de réalisation) en prévoyant un lien permanent, le câble, entre le drone et la centrale. De préférence dans ce cas, le drone est capable d'effectuer un vol sensiblement stationnaire, ce qui lui permet de rester positionné sensiblement au-dessus de la centrale pendant la phase d'acquisition. Le drone peut par exemple être un hélicoptère.

### Déplacements relatifs entre la structure de surface et le(s) capteur(s) et/ou système(s) de prélèvement d'échantillons

Différents agencements permettent d'assurer que la mesure ou le prélèvement est réalisé à la profondeur souhaitée :
Dans un premier mode de réalisation, la liaison mécanique comporte une entretoise fixée à la structure de surface, sur laquelle sont fixés une pluralité desdits au moins un capteur et/ou dudit au moins un système de prélèvement d'échantillons de telle sorte que ladite pluralité desdits au moins un capteur et/ou dudit au moins un système de prélèvement d'échantillons est positionnée auxdites profondeurs d'acquisition lorsque la structure de surface flotte à la surface du liquide pendant une phase d'acquisition.

Dans ce cas, le dispositif comporte autant de capteurs, et/ou de systèmes de prélèvement, qu'il y a de profondeurs d'acquisition auxquelles on veut procéder à une mesure ou à un prélèvement d'échantillon.

L'entretoise désigne une structure rigide, par exemple une tige métallique, sur laquelle sont fixés les capteurs et/ou les systèmes de prélèvement d'échantillons (ou, comme indiqué auparavant, les portions de détection des capteurs, et/ou les orifices de prélèvement des systèmes de prélèvement d'échantillons). L'entretoise peut ou non être fixée de manière rigide à la structure de surface ; elle peut par exemple être repliable, de manière à être repliée pendant les phases de transport aérien de la centrale d'acquisition.

Pendant les phases d'acquisition, l'entretoise doit être disposée de manière à placer les capteurs et/ou les systèmes de prélèvement d'échantillons aux profondeurs souhaitées.

Dans un deuxième mode de réalisation, le recours à de multiples capteurs et/ou systèmes de prélèvement d'échantillons est évité grâce à un 'ascenseur'. La liaison mécanique comporte en effet alors un ascenseur apte à déplacer ledit au moins un capteur et/ou ledit au moins un système de prélèvement d'échantillons à différentes profondeurs par rapport à la surface du liquide.

Un même capteur ou système de prélèvement d'échantillon est alors positionné successivement à différentes profondeurs pour réaliser les mesures ou prélèvements voulus. La phase d'acquisition se déroule par exemple de la manière suivante : Le capteur et /ou système de prélèvement est d'abord placé dans une position initiale, dans laquelle la profondeur du capteur et/ou du système de prélèvement est connue (elle découle de l'agencement de la centrale d'acquisition). Dans cette position le capteur ou système de prélèvement est par exemple sensiblement à la surface de l'eau. Ensuite le capteur et/ou système de prélèvement est descendu dans l'eau ; au cours de cette descente, la profondeur dudit capteur ou système de prélèvement est déterminée par rapport à la position initiale. Cette mesure peut se faire en lisant un index dont la valeur s'accroit au fur et à mesure que le capteur ou système de prélèvement descend.

Dans un mode de réalisation, l'ascenseur comporte un capteur de profondeur, lié au(x) capteur(s) et/ou au(x)dit(s) système(s) de prélèvement d'échantillons, et le dispositif est agencé de manière à utiliser l'information fournie par le capteur de profondeur pour positionner ledit au moins un capteur et/ou ledit au moins un système de prélèvement d'échantillons à une profondeur souhaitée. Le capteur de profondeur est naturellement agencé de manière à indiquer la profondeur à laquelle se trouve ledit au moins un capteur et/ou ledit au moins un système de prélèvement d'échantillons.

Les moyens de positionnement par exemple font descendre le capteur et/ou le système de prélèvement d'échantillon ; le capteur de profondeur est lié à l'un ou l'autre de ceux-ci, selon le cas. La mesure et/ou le prélèvement est déclenché lorsque le capteur de profondeur indique que la profondeur désirée est atteinte.

Dans un mode de réalisation, l'ascenseur comporte un treuil fixé sur la structure de surface de la centrale, apte à faire descendre le(s) capteur(s) et/ou le(s) système(s) de prélèvement d'échantillon retenu par un câble à la profondeur d'acquisition.

De préférence dans ce cas, le dispositif peut comporter un système de contrôle de la longueur de câble déroulé qui permet d'assurer que le capteur ou système de prélèvement se trouve pendant la mesure ou respectivement le prélèvement de l'échantillon à la profondeur d'acquisition prédéterminée voulue.

Dans un mode de réalisation, la centrale est apte à effectuer des mesures à l'aide dudit au moins un capteur, et le dispositif comporte des moyens d'enregistrement (14) des mesures effectuées, lesdits moyens d'enregistrement étant disposés à bord du drone, de la structure de surface de la centrale, ou dans un autre équipement fixe ou mobile apte à stocker des données. L'enregistrement des mesures effectuées peut ainsi notamment être fait par des moyens d'enregistrement disposés au sol, comprenant une unité d'enregistrement fixe agencée dans un immeuble. Pour alimenter l'unité d'enregistrement, la centrale d'acquisition (ou le drone) comporte pour sa part des moyens de transmission de données permettant la transmission des données à l'unité d'enregistrement.

Dans un mode de réalisation, le dispositif comporte des moyens de guidage du drone permettant le transport de la centrale d'un point à un autre sans intervention humaine. En d'autres termes, le drone est automatique, ou autoguidé. Le drone peut cependant également être un drone piloté à distance, notamment piloté à distance par un opérateur resté au sol, en particulier au moyen de commandes de haut-niveau. Pour faciliter le guidage du drone, le dispositif peut comporter des moyens de géolocalisation de la centrale d'acquisition, par exemple un GPS, une centrale inertielle ou autre. Ces moyens peuvent faire partie du drone et/ou de la centrale d'acquisition.

Dans un mode de réalisation, le dispositif est apte à réaliser ladite mesure et/ou ledit prélèvement d'échantillon, et plus généralement l'ensemble des opérations constituant d'une mission de mesure et/ou de prélèvement d'échantillons (dès lors que celle-ci a été programmée), de manière automatique, sans intervention humaine.

Le dispositif peut éventuellement être prévu pour réaliser d'autres mesures que celles relatives aux propriétés du liquide. Ainsi dans un mode de réalisation, le dispositif comporte en outre un appareil photo ou une caméra, aptes à prendre des photos pendant la phase d'acquisition, notamment des photos du liquide, en particulier dans le spectre visible, infrarouge, ou dans une partie de ces spectres.

L'invention sera bien comprise et ses avantages apparaîtront mieux à la lecture de la description détaillée qui suit, de modes de réalisation représentés à titre d'exemples non limitatifs. La description se réfère aux dessins annexés, sur lesquels :
- la figure 1 est une vue schématique en perspective représentant un dispositif d'acquisition dans un premier mode de réalisation de l'invention ;
- la figure 2 est une vue schématique en perspective représentant la centrale d'acquisition du dispositif d'acquisition de la figure 1 ;
- la figure 3 est une vue schématique de profil du dispositif d'acquisition de la figure 1 ;
- la figure 4 est une vue schématique en perspective représentant la sonde de mesure du dispositif d'acquisition de la figure 1 ;
- la figure 5 est une vue schématique en perspective représentant le système de prélèvement d'échantillons du dispositif d'acquisition de la figure 1 ; et
- la figure 6 est une vue schématique en perspective représentant un dispositif d'acquisition dans un second mode de réalisation de l'invention.

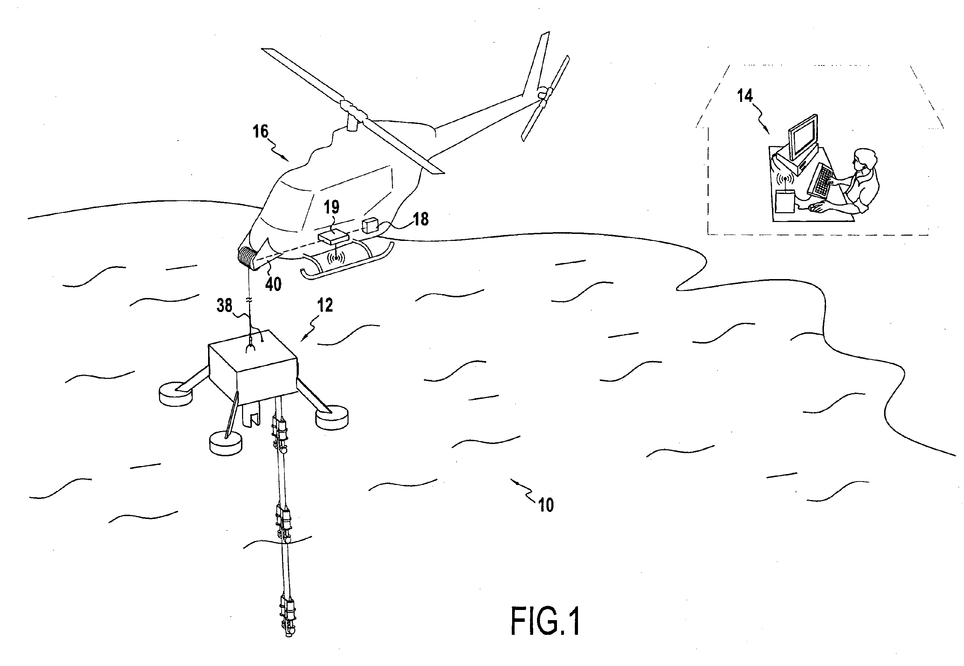

En faisant référence aux figures 1 à 5, un dispositif d'acquisition 10 selon l'invention va maintenant être décrit.

Ce dispositif d'acquisition 10 est un dispositif servant à réaliser des mesures de propriétés de l'eau et des prélèvements d'échantillons d'eau sur des plans d'eau. Il comprend une centrale d'acquisition automatique 12, une station de supervision 14 servant également d'unité de stockage d'informations, un drone volant 16, et un dispositif de flotteur 35.

La centrale d'acquisition 12 permet la réalisation de mesure et le prélèvement d'échantillons. Elle permet de transmettre les résultats des mesures effectuées à la station de supervision 14.

La station de supervision 14 est un poste de travail comprenant un calculateur, des moyens de transmission sans fil pour échanger des informations avec le drone 16, et une interface utilisateur. Elle permet la programmation et la supervision du drone (respectivement avant et pendant ses sorties), ainsi que l'enregistrement et la consultation des résultats des mesures effectuées par la centrale d'acquisition 12. Naturellement, dans un autre mode de réalisation la programmation/supervision du drone et l'enregistrement des résultats de mesure peuvent être réalisés depuis deux stations distinctes.

Le drone volant 16 est un drone de type hélicoptère autoguidé. Il comporte un système de géolocalisation (ou GPS) 18 qui permet à tout moment de connaître sa position. L'hélicoptère est un hélicoptère programmable comportant un calculateur embarqué 19. L'hélicoptère peut ainsi être programmé depuis la station de supervision 14 pour réaliser une mission comportant des phases de transport et des phases d'acquisition. L'ensemble de ces phases peut être programmé à l'avance. Aussi, lorsqu'une mission d'acquisition de mesures et de prélèvement d'échantillons est programmée, elle peut être lancée puis se dérouler de manière complètement automatique, sans intervention humaine. L'hélicoptère peut ainsi être programmé pour se déplacer d'un point d'acquisition ou point de mesure/prélèvement (c'est-à-dire un point auquel la centrale d'acquisition réalise les mesures et prélèvements d'échantillons prévus) à un autre.

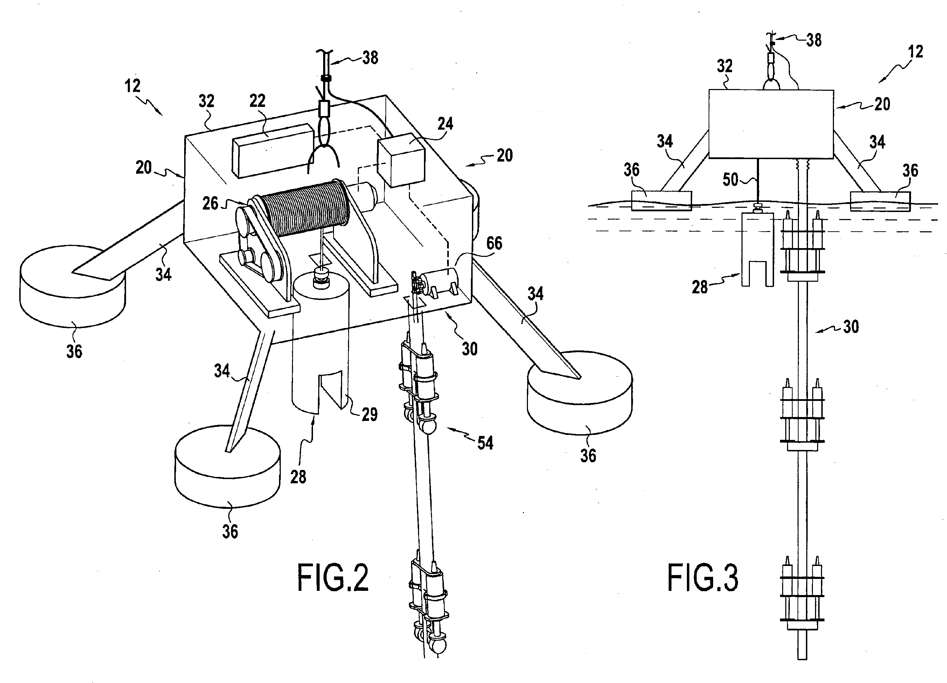

La centrale d'acquisition 12 ou unité d'acquisition 12 est illustrée par la figure 2. Elle comporte une structure de surface 20, une batterie 22, une unité de commande 24, un treuil 26, une sonde de mesure 28 (comprenant des capteurs qui seront décrits plus loin) et un système de prélèvement d'échantillons 30.

La structure de surface 20 est constitué par un boîtier sensiblement étanche 32 de forme générale rectangulaire, à l'intérieur duquel se trouvent la batterie 22, l'unité de commande 24, le treuil 26 et une partie du système de prélèvement d'échantillons 30. La structure 20 est supportée un dispositif de flotteur 35. Celui-ci comporte quatre pieds 34, fixés aux quatre coins inférieurs du boîtier 32. Une extrémité de chaque pied 34 est fixée au boîtier 32 alors que l'autre extrémité est liée à un flotteur 36. Les masses et volumes des flotteurs 36 sont déterminés de telle sorte que les flotteurs 36 permettent le maintien de la centrale 12 en position stable et fixe à la surface de l'eau, avec le boîtier 32 positionné au-dessus de la surface de l'eau (figure 3).

La batterie 22 est reliée à l'unité de commande 24, au treuil 26, à la sonde de mesure 28 et au système de prélèvement d'échantillons 30 et assure leur alimentation.

L'unité de commande 24 est reliée au treuil 26, à la sonde de mesure 28 et au système de prélèvement d'échantillons 30. Elle transmet à la sonde de mesure 28 les signaux de commande déclenchant la réalisation des mesures et collecte les valeurs des mesures effectuées. Elle commande le fonctionnement du système de prélèvement 30. Elle reçoit de l'hélicoptère via un câble 38 les informations de positions qu'elle associe aux résultats de mesure collectés.

La centrale 12 est reliée à l'hélicoptère 16 par le câble 38. Ce câble se déroule à partir d'un treuil 40 fixé sur le fuselage de l'hélicoptère 16. Il est à la fois un câble de support, qui permet de descendre ou remonter la centrale 12 de l'hélicoptère 16, et un câble de communication entre l'hélicoptère 16 et la centrale 12. Ainsi, pendant les phases d'acquisition, la centrale 12 est désolidarisée de l'hélicoptère 16 et descendue à la surface de l'eau. Elle n'est alors reliée à l'hélicoptère 16 que par le câble 38.

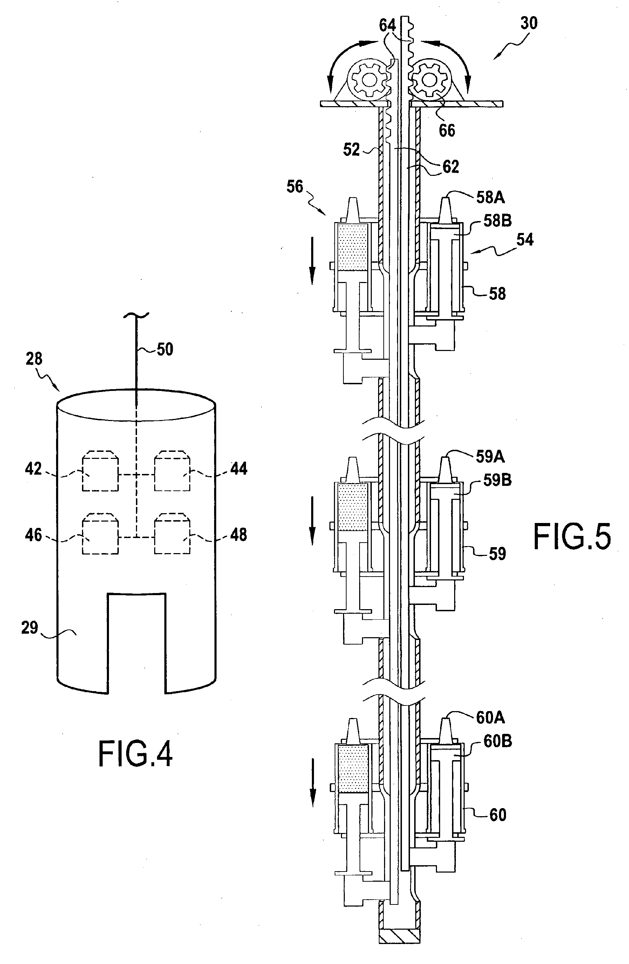

La sonde de mesure 28 comporte un boîtier de protection 29 qui seul est visible sur les figures 1 à 3. La partie active de la sonde 28 est située à l'intérieur de ce boîtier 29 et est illustrée par la figure 4. Elle comporte quatre capteurs ainsi qu'un micro-contrôleur. Ces capteurs sont respectivement un capteur de température 42, un capteur de pression 44, un capteur de conductivité 46 et un capteur de turbidité 48.

La sonde 28 est déplacée par rapport à la surface de l'eau, de manière à être positionnée aux différentes profondeurs d'acquisition, de la manière suivante :
La sonde est reliée à la structure de surface 20 par un câble 50. Ce câble 50 sert à la fois à soutenir la sonde 28 lorsqu'elle est descendue dans l'eau, et à permettre l'échange d'informations et de courant entre la sonde 28 et respectivement l'unité de commande 24 et la batterie 22. Les informations sont transmises par l'intermédiaire d'un coupleur optoélectronique placé sur l'axe de rotation du treuil, ce qui avantageusement permet d'éviter le recours à un contacteur tournant. Le câble 50 est enroulé sur le treuil 26, que commande l'unité de commande 24. Le déroulement du câble permet de faire descendre dans l'eau et de positionner la sonde de mesure aux différentes profondeurs d'acquisition choisies. Ainsi, le treuil 26 et le câble 50 assurent une liaison mécanique entre la structure de surface 20 et la sonde 28 qui constitue un ascenseur au sens de l'invention.

D'autre part, pour permettre le prélèvement d'échantillons aux différentes profondeurs d'acquisition, le système de prélèvement d'échantillon 30 n'est pas agencé avec un ascenseur mais avec une entretoise 52.

En effet, le système de prélèvement d'échantillon 30 (fig.5) comporte une entretoise, réalisée sous la forme d'une tige creuse verticale 52, qui est fixée rigidement à la face inférieure du boîtier 32. Celle-ci constitue une liaison mécanique entre la structure de surface 20, et le capteur 28 et le système 30 de prélèvement d'échantillons. La longueur de la tige 52 est fixée de telle sorte que la tige s'enfonce dans l'eau sur une profondeur d'au moins un mètre lorsque la centrale 12 flotte à la surface de l'eau. Autour de la tige 52 sont fixés des groupes de seringues. Sur la figure 2, seuls deux groupes 54, 56 de seringues sont représentés ; mais de manière générale, le nombre de groupes de seringues doit être égal (ou supérieur) au nombre de points en lesquels on souhaite procéder à des mesures et des prélèvements d'échantillons. Les groupes 54 et 56 sont identiques ; aussi seul le groupe 54 va être décrit dans ce qui suit.

Les seringues constituent des échantillonneurs ou équipements de prélèvement d'échantillon. Chaque seringue comprend en effet de manière bien connue un corps cylindrique dans laquelle un piston peut être déplacé de manière à remplir la seringue, le contenu de la seringue constituant l'échantillon d'eau prélevé par le dispositif.

Le groupe 54 comporte trois seringues identiques 58, 59 et 60. Ces seringues 58, 59, 60 sont fixées sur la tige 52 de manière à ce que leurs orifices respectifs 58A, 59A et 60A (orifices de prélèvement) se trouvent immergés à des profondeurs respectives de 10cm, 50cm et 1mètre lorsque la centrale 12 flotte à la surface de l'eau : Ces profondeurs sont les profondeurs d'acquisition. Les seringues 58, 59, 60 comportent respectivement chacune un piston 58B, 59B et 60B. Ces pistons sont reliés à une tige interne 62 qui peut coulisser à l'intérieur de la tige 52. Le sommet de la tige 62 comporte une crémaillère 64 qui engrène un pignon de l'arbre de sortie d'un moteur 66. La rotation du moteur 66 est commandée par l'unité de commande 24.

Sur la figure 5, le groupe de seringues 54 est représenté en position initiale (seringues vides), alors que le groupe de seringues 56 est représenté en position finale (seringues emplies). On voit sur la partie de droite de la figure 5 qu'en position initiale, la tige 62 est en position haute. Le remplissage des seringues 58, 59 et 60 est déclenché en actionnant le moteur 66. L'arbre de sortie de celui-ci se met à tourner ce qui fait descendre la tige 62. Chacun des pistons 58B, 59B et 60B est alors entraîné par la tige 62 et descend également, ce qui provoque le remplissage des seringues 58, 59 et 60 et le prélèvement d'échantillons d'eau dans celles-ci. La position finale est représentée sur la partie gauche de la figure 5, qui représente le groupe de seringues 56 après prélèvement des échantillons d'eau. Du fait de la position des seringues, leurs orifices de prélèvement étant aux profondeurs choisies de 10 cm, 50 cm et 1 mètre, les échantillons sont constitués par de l'eau se trouvant à ces profondeurs respectives.

Le fonctionnement du dispositif 10 va maintenant être décrit.

Le dispositif est utilisé pour assurer le suivi de la qualité des eaux d'un ensemble de plans d'eau répartis sur une zone accessible par l'hélicoptère 16, compte tenu de son rayon d'action. Pour assurer ce suivi, il convient dans un premier temps de programmer une mission type du dispositif d'acquisition 10. Cette mission consiste à effectuer des mesures et prélever des échantillons en un certain nombre de points de mesure. On suppose par exemple que l'on veut simplement faire un tel suivi en deux points de mesure situés respectivement sur le lac A et le lac B.

La programmation de la mission consiste à programmer depuis la station de supervision 14 les différents phases de déplacement et d'arrêt de l'hélicoptère 16, avec notamment pour la mission présentée ici trois déplacements, respectivement depuis son point de départ jusqu'au lac A, puis du lac A au lac B, et enfin le retour du lac B à son point de départ. Entre les phases de déplacement sont programmées au lac A et au lac B des phases d'acquisition. Le contenu des phases d'acquisition (c'est-à-dire la liste des mesures à effectuer et des échantillons à prélever) peut varier d'un point de mesure/prélèvement d'échantillons à un autre.

Une fois cette programmation effectuée, les missions peuvent être réalisées à la fréquence souhaitée. Les missions se déroulent de la manière suivante : Conformément au plan de vol préétabli, l'hélicoptère 16 transporte la centrale d'acquisition successivement aux différents points de mesure/prélèvement d'échantillons. En chacun de ces points, la centrale d'acquisition 12 est descendue au bout du câble 38 déroulé par le treuil 40 jusqu'à se poser sur la surface de l'eau. Pendant la phase d'acquisition, l'hélicoptère 16 reste en vol stationnaire au-dessus de la centrale 12, le câble 28 étant relâché, ce qui permet à la centrale 12 de pouvoir rester fixe et immobile à la surface de l'eau. L'hélicoptère 16 transmet via le câble 38 à l'unité de commande 24 la liste des mesures et prélèvements d'échantillons à réaliser, conformément à ce qui a été programmé.

Au sein de la centrale 12, l'unité de commande 24 pilote alors les différentes mesures et prélèvements d'échantillons. Par exemple, l'unité de commande 24 actionne le moteur 66 de manière à remplir les seringues 58, 59 et 60 : Ainsi sont prélevés trois échantillons, représentatifs de l'eau aux différentes profondeurs d'acquisition voulues, à savoir 10 cm, 50 cm et 1 mètre. En parallèle, le treuil 26 déroule le câble 50, jusqu'à ce qu'une chute de la tension du câble 50 indique que la sonde a atteint le fond du plan d'eau. A ce moment, l'unité de commande 24 déclenche une première acquisition des mesures, et commande l'acquisition d'une mesure de pression qui permet immédiatement de connaître la profondeur atteinte. L'unité de commande 24 commande alors le déplacement de la sonde vers le haut. Pendant cette remontée, le capteur de pression 46 est utilisé pour déterminer la pression, et donc la profondeur, en continu. Sur le fondement de cette information, l'unité de commande 24 envoie un signal de demande de mesure aux capteurs 42, 46 et 48 via le micro-contrôleur pour toutes les profondeurs atteintes multiples de 10 cm. Par suite, les capteurs 42, 46 et 48 réalisent une mesure tous les 10 cm au cours de la remontée de la sonde 28. L'unité de commande 24, pilotant le treuil 26 qui enroule le câble 50, en fonction des informations de pression reçues du capteur 44, permet de positionner les capteurs 42, 46 et 48 aux différentes profondeurs choisies.

Dans un mode de réalisation alternatif, au lieu que la sonde 28 ne comporte un capteur de pression, le treuil 26 pourrait comporter un compteur indexé, par exemple comprenant une piste potentiométrique, apte à mesurer la longueur de câble 50 déroulé. La profondeur atteinte par la sonde 28 serait alors déterminée à partir de la longueur de câble déroulée.

Une fois effectuées les opérations de mesure et les prélèvements d'échantillons, l'unité de commande 24 transmet à l'hélicoptère 16 un signal de fin de phase d'acquisition, accompagné de l'ensemble des résultats des mesures effectuées. Ces informations sont transmises immédiatement par l'hélicoptère 16 à la station de supervision 14 par liaison sans fil. Ces informations sont alors enregistrées et stockées par la station de supervision 14.

De manière alternative, les résultats de mesure pourraient aussi être directement transmis par la centrale d'acquisition à la station de supervision 14, sans transiter par l'hélicoptère 16.
(Naturellement, ces informations peuvent de manière alternative et éventuellement complémentaire, être aussi stockées soit par la centrale d'acquisition 12 elle-même, soit par le calculateur embarqué de l'hélicoptère 16).

A réception du signal de fin de phase d'acquisition, le calculateur 19 de l'hélicoptère commande l'enroulement du câble 28 par le treuil 40 de manière à faire remonter la centrale d'acquisition 12 jusqu'au contact de l'hélicoptère 16. Lorsque la centrale 12 a été entièrement remontée et se retrouve ainsi à nouveau solidement fixée à l'hélicoptère, le calculateur 19 lance la phase de déplacement suivante programmée.

Lorsque l'hélicoptère a parcouru l'ensemble des points de mesure/prélèvements programmés, il retourne à son point de départ. Les échantillons sont alors récupérés, et les résultats de mesure sont extraits de la station de supervision 14, afin de permettre de réaliser le suivi de qualité des eaux aux différents points de mesure.

Dans ce mode de réalisation, la centrale d'acquisition 12 reste reliée à l'hélicoptère 16 par un câble (38). Dans un mode de réalisation alternatif non représenté, la centrale d'acquisition est fixée à l'hélicoptère, pendant les phases de transport, par un électro-aimant fixé à l'extrémité du câble 38. Le câble 38 est alors seulement un câble de support et ne permet pas la transmission d'informations. La récupération de la centrale d'acquisition à l'issue d'une phase d'acquisition se passe alors de la manière suivante : L'hélicoptère se positionne en vol stationnaire juste au-dessus de la centrale d'acquisition. L'électro-aimant est alors activé ; le câble 38 est déroulé par le treuil 40 jusqu'à ce que le contact soit établi entre la partie supérieure de la centrale d'acquisition, sur laquelle est fixée une masse ferreuse attirée par l'électro-aimant, et ce dernier. L'ensemble (électro-aimant et centrale d'acquisition) est alors remonté à bord de l'hélicoptère par le treuil. Dans ce mode de réalisation, les communications entre l'hélicoptère et la centrale d'acquisition se font sans fil. Aussi pendant la phase d'acquisition, la centrale est entièrement détachée (sans aucune liaison mécanique) de l'hélicoptère.

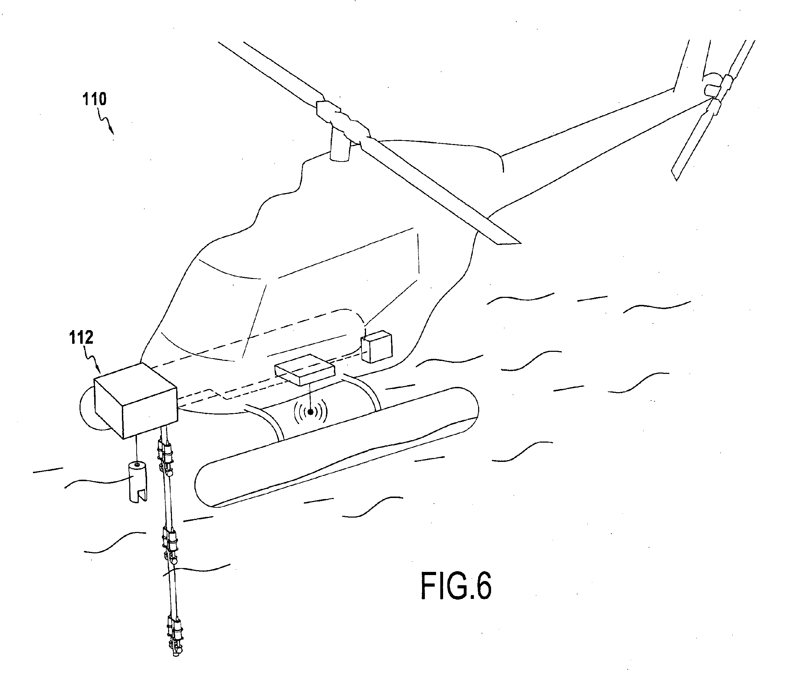

La figure 6 représente un dispositif d'acquisition 110 dans un second mode de réalisation de l'invention.

Ce dispositif est très semblable au dispositif 10 aussi seules les différences entre ces dispositifs seront explicitées.

Dans le dispositif 110, la centrale d'acquisition 112 au lieu de pouvoir être désolidarisée de l'hélicoptère et descendue à la surface de l'eau au bout d'un câble, est fixée rigidement au fuselage de l'hélicoptère. La centrale d'acquisition 112 est identique à la centrale 12, hormis le fait qu'elle ne comporte pas de pieds et est fixée à l'hélicoptère. La centrale 112 est fixée à l'hélicoptère de manière à se trouver, lorsque l'hélicoptère a amerri et flotte à la surface de l'eau, dans la même position relative par rapport à l'eau que la centrale 12. Par suite, les opérations de mesure et de prélèvement d'échantillons effectuées par la centrale d'acquisition 112 sont identiques à celles réalisées par la centrale 12 du dispositif 10.

L'hélicoptère 116 assure le maintien de la centrale d'acquisition 112 au dessus de la surface de l'eau. Aussi dans ce mode de réalisation, il constitue le dispositif de flotteur au sens de l'invention.

Bien que cela ne soit pas représenté, dans les deux modes de réalisation le dispositif d'acquisition comporte des moyens pour rehausser la centrale d'acquisition par rapport à l'hélicoptère lors des phases d'atterrissage. Ces moyens permettent de faire en sorte que lors des atterrissages de l'hélicoptère sur le sol, les parties inférieures de la centrale d'acquisition (notamment le système 30 de prélèvement d'échantillon, et la sonde de mesure 28) ne touchent le sol et ne soient endommagées.

Dans les deux modes de réalisation décrits précédemment, le GPS (18) est embarqué à bord du drone. On notera cependant que dans d'autres modes de réalisation de l'invention, les moyens de positionnement tels que le GPS peuvent être intégrés non pas au drone, mais à la centrale d'acquisition elle-même. Cela permet d'obtenir des informations de position plus précises en ce qui concerne l'emplacement des mesures et/ou des prélèvement d'échantillons effectués.

Enfin, bien que les différents modes de réalisation présentés aient consisté en des dispositifs entièrement automatiques, ne nécessitant aucune intervention humaine pour la réalisation des missions de mesure et de prélèvements d'échantillons (c'est-à-dire, une fois que la programmation initiale a été faite, et à l'exclusion éventuellement de la préparation du dispositif avant son envol), l'invention couvre également l'ensemble des modes de réalisation dans lesquels tout ou partie de ces différentes opérations sont télécommandées par un opérateur (humain), opérant notamment à partir d'une station de supervision analogue à la station de supervision 14 du dispositif 10.

## Revendications

1. Dispositif d'acquisition (16, 116) comprenant :
- une centrale d'acquisition (12,112), comportant :
. une structure dite structure de surface (20) ;
. au moins un capteur (42,44,46,48) et/ou au moins un système (30) de prélèvement d'échantillon ; et
. une liaison mécanique (26,52) entre ledit au moins un capteur et/ou ledit au moins un système de prélèvement et la structure de surface, agencée de manière à permettre de positionner ledit au moins un capteur et/ou ledit au moins un système de prélèvement à une pluralité de profondeurs dites profondeurs d'acquisition prédéterminées par rapport à une surface d'un liquide ;
- un drone volant (16), apte à transporter la centrale d'un point à un autre ;
- un dispositif de flotteur (35,116) apte à maintenir ladite structure de surface en position sensiblement fixe par rapport à la surface du liquide, au moins en partie au-dessus de ladite surface ;
le dispositif étant apte à réaliser une phase d'acquisition pendant laquelle la structure de surface est ainsi maintenue en position sensiblement fixe par rapport à la surface du liquide, et une même mesure d'une propriété relative audit liquide est effectuée par ledit au moins un capteur, et/ou au moins un échantillon du liquide est prélevé par ledit au moins un système de prélèvement d'échantillon, à chacune desdites profondeurs d'acquisition.

2. Dispositif d'acquisition selon la revendication 1, dans lequel ladite liaison mécanique comporte un ascenseur (26,50) apte à déplacer ledit au moins un capteur et/ou ledit au moins un système de prélèvement d'échantillons à différentes profondeurs par rapport à ladite surface du liquide.

3. Dispositif d'acquisition selon la revendication 2, dans lequel l'ascenseur comporte en outre un capteur de profondeur (44), lié audit au moins un capteur et/ou audit au moins un système de prélèvement d'échantillons, le dispositif étant agencé de manière à utiliser l'information fournie par ledit capteur de profondeur pour positionner ledit au moins un capteur et/ou ledit au moins un système de prélèvement d'échantillons à une profondeur souhaitée.

4. Dispositif d'acquisition selon la revendication 2 ou 3, dans lequel ledit ascenseur comporte un treuil (26) fixé sur la structure de surface de la centrale, apte à faire descendre ledit au moins un capteur (42,44,46,48) et/ou ledit au moins un système de prélèvement d'échantillon retenu par un câble (50) à la profondeur d'acquisition.

5. Dispositif d'acquisition selon la revendication 4, dans lequel le dispositif comporte un système de contrôle de la longueur de câble déroulé qui permet d'assurer que le capteur ou l'orifice de prélèvement se trouve pendant la mesure ou respectivement le prélèvement de l'échantillon à la profondeur d'acquisition prédéterminée voulue.

6. Dispositif d'acquisition selon l'une quelconque des revendications 1 à 5, dans lequel la centrale est apte à effectuer des mesures à l'aide dudit au moins un capteur, et le dispositif comporte des moyens d'enregistrement (14) des mesures effectuées, lesdits moyens d'enregistrement étant disposés à bord du drone, de la structure de surface, ou dans un autre équipement fixe ou mobile apte à stocker des données.

7. Dispositif d'acquisition selon l'une quelconque des revendications 1 à 6, dans lequel la structure de surface comporte en outre un boîtier étanche, et le dispositif de flotteur (36,16) est agencé de manière à maintenir ledit boîtier à distance au-dessus de la surface du liquide.

8. Dispositif d'acquisition selon l'une quelconque des revendications 1 à 7, le dispositif étant apte à réaliser ladite mesure et/ou ledit prélèvement d'échantillon de manière automatique, sans intervention humaine.

9. Dispositif d'acquisition selon l'une quelconque des revendications 1 à 8, dans lequel ladite liaison mécanique comporte en outre une entretoise (52) fixée à la structure de surface, sur laquelle sont fixés une pluralité desdits au moins un capteur et/ou dudit au moins un système de prélèvement d'échantillons de telle sorte que ladite pluralité desdits au moins un capteur et/ou dudit au moins un système de prélèvement d'échantillons est positionnée auxdites profondeurs d'acquisition lorsque la structure de surface flotte à la surface du liquide pendant une phase d'acquisition.

10. Dispositif d'acquisition selon l'une quelconque des revendications 1 à 9, dans lequel la centrale d'acquisition (112) est solidaire du drone et celui-ci est apte à amerrir pour permettre la réalisation de la mesure ou du prélèvement de l'échantillon.

11. Dispositif d'acquisition selon l'une quelconque des revendications 1 à 9, dans lequel la centrale d'acquisition est apte à être totalement détachée du drone pendant la phase d'acquisition.

## Patentansprüche

1. Erfassungsvorrichtung (16, 116), umfassend:
- eine Erfassungszentrale (12, 112), umfassend:
• eine Struktur, sogenannte Oberflächenstruktur (20),
• wenigstens einen Sensor (42, 44, 46, 48) und/oder wenigstens ein System (30) zur Probenentnahme, und
• eine mechanische Verbindung (26, 52) zwischen dem wenigstens einen Sensor und/oder dem wenigstens einen Entnahmesystem und der Oberflächenstruktur, die dazu eingerichtet ist, zu ermöglichen, den wenigstens einen Sensor und/oder das wenigstens eine Entnahmesystem in einer Vielzahl von Tiefen, sogenannten vorbestimmten Erfassungstiefen, in Bezug auf eine Oberfläche einer Flüssigkeit zu positionieren,
- eine fliegende Drohne (16), die geeignet ist, die Zentrale von einer Stelle zur nächsten zu transportieren,
- eine Schwimmervorrichtung (35, 116), die geeignet ist, die Oberflächenstruktur in im Wesentlichen fester Position gegenüber der Oberfläche der Flüssigkeit, wenigstens teilweise oberhalb der Oberfläche zu halten,
wobei die Vorrichtung geeignet ist, eine Erfassungsphase durchzuführen, während derer die Oberflächenstruktur somit in im Wesentlichen fester Position gegenüber der Oberfläche der Flüssigkeit gehalten wird und eine gleiche Messung einer Eigenschaft bezüglich der Flüssigkeit durch den wenigstens einen Sensor durchgeführt wird und/oder wenigstens eine Probe der Flüssigkeit durch das wenigstens eine Probenentnahmesystem in jeder der Erfassungstiefen entnommen wird.

2. Erfassungsvorrichtung nach Anspruch 1, wobei die mechanische Verbindung einen Aufzug (26, 50) umfasst, der geeignet ist, den wenigstens einen Sensor und/oder das wenigstens eine Probenentnahmesystem in unterschiedlichen Tiefen in Bezug auf die Oberfläche der Flüssigkeit zu bewegen.

3. Erfassungsvorrichtung nach Anspruch 2, wobei der Aufzug ferner einen Tiefensensor (44), der mit dem wenigstens einen Sensor und/oder mit dem wenigstens einen Probenentnahmesystem verbunden ist, umfasst, wobei die Vorrichtung eingerichtet ist, um die durch den Tiefensensor gelieferte Information zu verwenden, um den wenigstens einen Sensor und/oder das wenigstens eine Probenentnahmesystem in einer gewünschten Tiefe zu positionieren.

4. Erfassungsvorrichtung nach Anspruch 2 oder 3, wobei der Aufzug eine an der Oberflächenstruktur der Zentrale befestigte Winde (26) umfasst, die geeignet ist, den wenigstens einen Sensor (42, 44, 46, 48) und/oder das wenigstens eine Probenentnahmesystem, das durch ein Kabel (50) gehalten ist, in die Erfassungstiefe hinabzulassen.

5. Erfassungsvorrichtung nach Anspruch 4, wobei die Vorrichtung ein System zum Steuern der Länge von abgewickeltem Kabel umfasst, das ermöglicht, sicherzustellen, dass der Sensor oder die Entnahmeöffnung sich während der Messung bzw. der Entnahme der Probe in der gewünschten vorbestimmten Erfassungstiefe befindet.

6. Erfassungsvorrichtung nach einem der Ansprüche 1 bis 5, wobei die Zentrale geeignet ist, Messungen mit Hilfe des wenigstens einen Sensors durchzuführen, und die Vorrichtung Mittel zum Aufzeichnen (14) der durchgeführten Messungen umfasst, wobei die Aufzeichnungsmittel an Bord der Drohne, der Oberflächenstruktur oder in einer anderen festen oder mobilen Einrichtung, welche geeignet ist, Daten zu speichern, angeordnet sind.

7. Erfassungsvorrichtung nach einem der Ansprüche 1 bis 6, wobei die Oberflächenstruktur ferner ein dichtes Gehäuse umfasst und die Schwimmervorrichtung (36, 16) eingerichtet ist, um das Gehäuse im Abstand oberhalb der Oberfläche der Flüssigkeit zu halten.

8. Erfassungsvorrichtung nach einem der Ansprüche 1 bis 7, wobei die Vorrichtung geeignet ist, die Messung und/oder die Probenentnahme automatisch, ohne menschliches Eingreifen durchzuführen.

9. Erfassungsvorrichtung nach einem der Ansprüche 1 bis 8, wobei die mechanische Verbindung ferner einen an der Oberflächenstruktur befestigten Steg (52) umfasst, an dem eine Vielzahl von dem wenigstens einen Sensor und/oder von dem wenigstens einen Probenentnahmesystem derart befestigt ist, dass die Vielzahl von dem wenigstens einen Sensor und/oder von dem wenigstens einen Probenentnahmesystem in den Erfassungstiefen positioniert ist, wenn die Oberflächenstruktur während einer Erfassungsphase an der Oberfläche der Flüssigkeit schwimmt.

10. Erfassungsvorrichtung nach einem der Ansprüche 1 bis 9, wobei die Erfassungszentrale (112) mit der Drohne fest verbunden ist und diese geeignet ist, zu wassern, um die Durchführung der Messung oder der Probenentnahme zu ermöglichen.

11. Erfassungsvorrichtung nach einem der Ansprüche 1 bis 9, wobei die Erfassungszentrale geeignet ist, während der Erfassungsphase von der Drohne vollständig abgenommen zu werden.

## Claims

1. An acquisition device (16, 116) comprising:
• an acquisition unit (12, 112), comprising:
• a structure referred to as a surface structure (20);
• at least one sensor (42, 44, 46, 48) and/or at least one sample-taking system (30); and
• a mechanical connection (26, 52) between the surface structure and said at least one sensor and/or said at least one sample-taking system, the mechanical connection being arranged in such a manner as to enable said at least one sensor and/or said at least one sample-taking system to be positioned at a plurality of depths referred to as acquisition depths that are predetermined relative to a surface of a liquid;
• an aerial drone (16) suitable for transporting the unit from one point to another; and
• a float device (35, 116) suitable for holding said surface structure in a position that is substantially stationary relative to the surface of the liquid, and at least in part above said surface;
the device being suitable for performing an acquisition stage during which the surface structure is thus held in a position that is substantially stationary relative to the surface of the liquid, and the same measurement of a property relating to said liquid is performed by said at least one sensor, and/or at least one sample of liquid is taken by said at least one sample-taking system, at each of said acquisition depths.

2. An acquisition device according to claim 1, wherein said mechanical connection comprises a hoist (26, 50) suitable for moving said at least one sensor and/or said at least one sample-taking system to different depths relative to said surface of the liquid.

3. An acquisition device according to claim 2, wherein the hoist further comprises a depth sensor (44) connected to said at least one sensor and/or to said at least one sample-taking system, the device being arranged in such a manner as to use the information supplied by said depth sensor in order to position said at least one sensor and/or said at least one sample-taking system at a desired depth.

4. An acquisition device according to claim 2 or claim 3, wherein said hoist comprises a winch (26) fastened to the surface structure of the unit and suitable for lowering to the acquisition depth said at least one sensor (42, 44, 46, 48) and/or said at least one sample-taking system held by a cable (50).

5. An acquisition device according to claim 4, wherein the device includes a control system for controlling the length of the cable wound out that makes it possible to ensure that the sensor or the sample-taking orifice is located at the desired predetermined acquisition depth during a measurement or while taking a sample.

6. An acquisition device according to any one of claims 1 to 5, wherein the unit is suitable for taking measurements with the help of said at least one sensor, and the device includes recording means (14) for recording the measurements taken, said recording means being arranged on board the drone, the surface structure, or in other stationary or movable equipment suitable for storing data.

7. An acquisition device according to any one of claims 1 to 6, wherein the surface structure further includes a leaktight housing, and the float device (36, 16) is arranged in such a manner so as to keep said housing at a distance above the surface of the liquid.

8. An acquisition device according to any one of claims 1 to 7, the device being suitable for taking said measurement and/or taking a sample in automatic manner, without human intervention.

9. An acquisition device according to any one of claims 1 to 8, wherein said mechanical connection further includes a spacer (52) fastened to the surface structure and having fastened thereon a plurality of said at least one sensor and/or of said at least one sample-taking system in such a manner that said plurality of said at least one sensor and/or of said at least one sample-taking system is positioned at said acquisition depths when the surface structure is floating on the surface of the liquid during an acquisition stage.

10. An acquisition device according to any one of claims 1 to 9, wherein the acquisition unit (112) is secured to the drone and the drone is suitable for alighting on the liquid in order to take the measurement or to take a sample.

11. An acquisition device according to any one of claims 1 to 9, wherein the acquisition unit is suitable for being completely detached from the drone during the acquisition stage.
